Europäisches Patentamt

European Patent Office

Office européen des brevets

(11) Veröffentlichungsnummer: **0 000 910**
A1

# EUROPÄISCHE PATENTANMELDUNG

(21) Anmeldenummer: **78100656.4**

(22) Anmeldetag: **14.08.78**

(51) Int. Cl.²: **C 07 H 3/04**, C 07 H 3/02,
C 07 C 49/17, C 07 C 47/19,
C 07 C 47/10, C 07 C 31/18,
C 07 C 29/14, C 07 C 29/00
// C08G18/42, C08G18/48

---

(30) Priorität: **24.08.77 DE 2738154**

(43) Veröffentlichungstag der Anmeldung: **07.03.79**
**Patentblatt 79/5**

(84) Benannte Vertragsstaaten: **BE CH DE FR GB LU NL**

(71) Anmelder: **Bayer Aktiengesellschaft, Zentralbereich Patente,Marken und Lizenzen Bayerwerk, D-5090 Leverkusen 1 (DE)**

(72) Erfinder: **Möhring, Edgar, Dr., Huferweg 49a, D-5060 Bergisch-Gladbach 2 (DE)**
Erfinder: **Müller, Hanns Peter, Dr., Berta-von-Suttner-Strasse 40, D-5090 Leverkusen (DE)**
Erfinder: **Wagner, Kuno, Dr., Am Kiesberg 8, D-5090 Leverkusen (DE)**

---

(54) **Verfahren zur Herstellung von niedermolekularen Polyhydroxylverbindungen.**

(57) Verfahren zur Herstellung von niedermolekularen Polyhydroxylverbindungen durch Selbstkondensation von Formaldehydhydrat in Gegenwart von löslichen oder unlöslichen Verbindungen von Metallen der zweiten bis vierten Hauptgruppe oder der zweiten bis achten Nebengruppe des periodischen Systems der Elemente als Katalysator sowie gegebenenfalls in Gegenwart von zur Endiolbildung befähigten Verbindungen als Cokatalysator und/oder von niedermolekularen und/oder höhermolekularen Polyhydroxylverbindungen, dadurch gekennzeichnet, dass der pH-Wert während der Kondensationsreaktion durch die Zugabe von 5 bis 200 Milliäquivalenten eines tertiären Amins, bezogen auf 1 Mol Formaldehyd, gesteuert wird.

EP 0 000 910 A1

BAYER AKTIENGESELLSCHAFT          5090 Leverkusen, Bayerwerk
Zentralbereich                    Sft/bc
Patente, Marken und Lizenzen

Verfahren zur Herstellung von niedermolekularen Polyhydroxylverbindungen

Die vorliegende Erfindung betrifft ein verbessertes Verfahren zur Herstellung von Formose durch Kondensation von wäßrigem Formaldehyd. Die Verbesserung besteht darin, daß zur
Steuerung des pH-Wertes erfindungsgemäß anstelle der bisher üblichen anorganischen Basen tertiäre Amine eingesetzt
werden.

Unter "Formose" werden erfindungsgemäß die an sich bekannten
Gemische von niedermolekularen Polyhydroxyl-Verbindungen
(mehrwertigen Alkoholen, Hydroxyaldehyden und Hydroxyketonen)
verstanden, welche bei der Kondensation von Formaldehydhydrat entstehen.

Die Herstellung von Gemischen mehrwertiger Alkohole, Hydroxyaldehyde und Hydroxyketone durch Selbstkondensation des
Formaldehydhydrats wird in zahlreichen Literaturstellen
beschrieben. Beispielsweise seien in diesem Zusammenhang
Butlerow und Loew, Annalen 120, 295 (1861), bzw. J.pr.Chem.
33, 321 (1886), Pfeil, chemische Berichte 84, 229 (1951),
Pfeil und Schroth, chemische Berichte 85, 303 (1952), R.D.
Partridge und A.H. Weiss, Carbohydrate Research 24,

Le A 18 370

29-44 (1972), die Formosen aus Glycerinaldehyd bzw. Dioxyaceton nach Emil Fischer, die deutschen Patentschriften 822 385, 830 951 und 884 794, die US-Patentschriften 2 224 910, 2 269 935 und 2 272 378 sowie die englische Patentschrift 513 708 genannt. Diese bekannten Verfahren des Standes der Technik sind jedoch mit gewissen Nachteilen behaftet (toxikologisch bedenkliche Katalysatoren, schlechte Raum-Zeit-Ausbeuten, gefärbte Nebenprodukte); in jüngster Zeit wurden jedoch von der Anmelderin neue Verfahren entwickelt, nach denen sich in hoher Ausbeute mit gängigen Katalysatoren praktisch farblose und von störenden Nebenprodukten freie Formosen herstellen lassen.

Eines dieser neuen Verfahren besteht darin, daß man die Kondensation des Formaldehydhydrats in Gegenwart von löslichen oder unlöslichen Blei(II)-salzen, bzw. an hochmolekulare Träger gebundenen Blei(II)-Ionen als Katalysator und eines Gemisches aus Hydroxyaldehyden und Hydroxyketonen als Co-Katalysator ablaufen läßt, wie es bei der Kondensation von Formaldehydhydrat entsteht und welches durch folgende Molverhältnisse charakterisiert ist:

Verbindungen mit 3 C-Atomen/Verbindungen mit 4 C-Atomen:
0,5 bis 2,0
Verbindungen mit 4 C-Atomen/Verbindungen mit 5 C-Atomen:
0,2 bis 2,0
Verbindungen mit 5 C-Atomen/Verbindungen mit 6 C-Atomen:
0,5 bis 5,0
wobei der Anteil der Komponenten mit 3 bis 6 C-Atomen mindestens 75 Gew.-%, vorzugsweise mehr als 85 Gew.-%, bezogen auf gesamten Co-Katalysator, beträgt.

Die Reaktionstemperatur liegt dabei im allgemeinen zwischen 70 und 110°C, bevorzugt zwischen 80 und 100°C, und

Le A 18 370

der pH-Wert der Reaktionslösung wird durch kontrollierte Zugabe einer Base bis zu einem Umsatz von 10-60 %, vorzugsweise 30-50 %, auf einen Wert von 6,0-8,0, bevorzugt 6,5-7,0, und anschließend auf einen Wert von 4,0 - 6,0, bevorzugt 5,0 - 6,0, eingestellt.

Überraschenderweise läßt sich die Produktverteilung der entsprechenden Polyol-, Hydroxyaldehyd- und Hydroxyketongemische durch diese spezielle pH-Führung und durch anschließende Kühlung bei verschieden hohen Restformaldehydgehalten (0 bis 10 Gew.-%, vorzugsweise 0,5 bis 6 Gew.-%) in reproduzierbarer Weise variieren.

Nachdem man die Selbstkondensation des Formaldehydhydrats durch Kühlen und/oder durch Desaktivierung des bleihaltigen Katalysators mittels Säuren unterbrochen hat, wird gegebenenfalls der Katalysator in an sich bekannter Weise entfernt und das in den Produkten enthaltene Wasser verdampft. Hinsichtlich näherer Einzelheiten sei auf die deutsche Patentanmeldung P 2 639 084.1 verwiesen.

Eine weitere Möglichkeit, in hoher Raum-Zeit-Ausbeute hochkonzentrierte farblose Formosen herzustellen, besteht darin, wäßrige Formalinlösungen und/oder Paraformaldehyd-Dispersionen in Gegenwart eines löslichen oder unlöslichen Metallkatalysators und eines durch teilweise Oxidation eines zwei- oder mehrwertigen, mindestens zwei benachbarte Hydroxylgruppen aufweisenden Alkohols mit einem Molekulargewicht zwischen 62 und 242 bzw. eines Gemisches derartiger Alkohole dargestellten Co-Katalysators zu kondensieren, wobei man den pH-Wert der Reaktionslösung durch gesteuerte Zufuhr einer Base bis zu einem Umsatz von 5 bis 40 % zwischen 6,0 und 9,0 hält und anschließend bis zum Abbruch der Kondensations-

Le A 18 370

reaktion auf 4,5 bis 8,0 einstellt, so daß er nun um 1,0 bis 2,0 Einheiten tiefer liegt als in der ersten Reaktionsphase, dann die Reaktion bei einem Restgehalt von 0-10 Gew.-% Formaldehyd durch Desaktivierung des Katalysators unterbricht und den Katalysator entfernt. Im Detail wird diese Arbeitsweise in der deutschen Patentanmeldung P 2 714 084.7 beschrieben.

Qualitativ hochwertige Formosen können auch durch Kondensation von Formaldehyd in Gegenwart eines Metallkatalysators und mehr als 10 Gew.-%, bezogen auf Formaldehyd, eines oder mehrerer zwei- oder mehrwertiger niedermolekularer Alkohole und/oder höhermolekularer Polyhydroxylverbindungen hergestellt werden. Derartige Formose-Polyolgemische sind Gegenstand der deutschen Patentanmeldung P 2 714 104.4.

Besonders wirtschaftlich ist es, Formose direkt, d.h. ohne den Umweg über wäßrige Formalinlösungen oder Paraformaldehyd, aus Formaldehyd enthaltenden Synthesegasen herzustellen. Man leitet zu diesem Zweck die Synthesegase, wie sie bei der großtechnischen Herstellung von Formaldehyd anfallen, bei Temperaturen zwischen 10 und 150°C kontinuierlich oder diskontinuierlich in eine Absorptionsflüssigkeit, welche aus Wasser, ein- oder mehrwertigen niedermolekularen Alkoholen und/oder höhermolekularen Polyhydroxylverbindungen und/oder zur Endiolbildung befähigten Verbindungen als Co-Katalysator und/oder löslichen oder unlöslichen Metallverbindungen, welche gegebenenfalls an hochmolekulare Träger gebunden sind, als Katalysator besteht und einen pH-Wert von 3 bis 10 aufweist, kondensiert den Formaldehyd direkt in situ in der Absorptionsflüssigkeit (gegebenenfalls auch in einem

Le A 18 370

nachgeschalteten Reaktionsrohr bzw. einer nachgeschalteten
Rührkesselkaskade), bricht die Selbstkondensation des
Formaldehyds bei einem Restformaldehydgehalt im Reaktionsgemisch von 0 bis 10 Gew.-% durch Kühlen und/oder durch
Desaktivierung des Katalysators mittels Säuren ab und entfernt schließlich den Katalysator. Bezüglich näherer
Einzelheiten dieses Verfahrens sei auf die deutsche Patentanmeldung P 2 721 093.1 verwiesen.

Die so hergestellten Formosen können nachträglich auch durch
überschüssigen Formaldehyd in ihre Halbacetale übergeführt
oder durch Reaktion mit Formaldehyd in Gegenwart von Basen
$\alpha$-methyloliert werden.

Je nach der Reaktionsführung bei der Formaldehydkondensation
lassen sich die Eigenschaften der Formose in weiten Grenzen
variieren. Im allgemeinen ist das mittlere Molekulargewicht
und damit die Hydroxylfunktionalität der Formosen um so
höher, je weiter die Kondensationsreaktion geführt wird,
d.h. je weniger Restformaldehyd beim Abbruch der Kondensationsreaktion bis zu einem Restformaldehydgehalt von 0
bis 1,5 Gew.-% geführt wird, eine Formose erhalten, welche
ca. 25 Gew.-% an Anteilen mit 5 C-Atomen, 45 Gew.-% an Verbindungen mit 6 C-Atomen und ca. 20 Gew.-% an Verbindungen
mit 7 und mehr C-Atomen enthält. Dagegen werden zusammen
nur ca. 10 % an Polyolen, Hydroxyketonen und Hydroxyaldehyden mit 2,3 und 4 C-Atomen erhalten. Dies entspricht
einer mittleren Hydroxylfunktionalität von ca. 5.

Le A 18 370

Durch Abbruch der Formaldehydselbstkondensation bei etwas höheren Restformaldehydgehalten werden, wie oben erläutert, andere Komponentenverteilungen der Startergemische erhalten. So ergibt sich bei einem Abbruch der Kondensationsreaktion bei 2 bis 2,5 % Formaldehydgehalt ein Gemisch mehrwertiger Alkohole, Hydroxyaldehyde und Hydroxyketone mit einer mittleren Hydroxylfunktionalität von ca. 4. Noch andere Komponentenverteilungen mit weiter erniedrigter durchschnittlicher Hydroxylfunktionalität werden erhalten, wenn man die Kondensationsreaktion bei Restformaldehydgehalten abbricht, die noch höher liegen als 2,5.

Bei all diesen Verfahren laufen neben der Selbstkondensation des Formaldehydhydrats in relativ hohem Maße auch gekreuzte Cannizzaro-Reaktionen zwischen Formaldehyd und den Carbonylgruppen der Formaldehyd-Kondensationsprodukte ab, was dazu führt, daß die so erhaltenen Formosen neben Hydroxyaldehyden und Hydroxyketonen eine erhebliche Menge an mehrwertigen Alkoholen enthalten. Dies kann für viele Zwecke von Vorteil sein, insbesondere dann, wenn ohnehin beabsichtigt ist, die Formose zu Gemischen mehrwertiger Alkohole zu hydrieren. Für andere Anwendungszwecke (beispielsweise die Herstellung spezieller schwer entflammbarer Polyurethanschaumstoffe oder die Verwendung der Formose als Substrat in Nährböden von Mikroorganismen) wäre es jedoch vorteilhaft, wenn die Formose einen möglichst hohen Anteil an reduzierenden Zuckern (d.h. möglichst wenig an durch gekreuzte Cannizzaro-Reaktion entstandenen mehrwertigen Alkoholen) enthält. Außerdem stören bei vielen Anwendungszwecken die bei der gekreuzten Cannizzaro-Reaktion entstandenen organischen Säuren (im wesentlichen Ameisen-

Le A 18 370

säure, daneben auch Spuren von Essigsäure, Milchsäure, Glykolsäure und Zuckersäuren), welche sich aus dem Reaktionsgemisch nur in einem technisch relativ aufwendigen Verfahrensschritt mittels Anionenaustauschern entfernen lassen.

Überraschenderweise wurde nunmehr gefunden, daß die gekreuzte Cannizzaro-Reaktion während der Formosebildung weitgehend unterdrückt wird, wenn man zur Einstellung des gewünschten pH-Wertes anstelle der bisher verwendeten anorganischen Basen (im allgemeinen Alkali- oder Erdalkalihydroxide) tertiäre Amine einsetzt. Im Gegensatz zu den anorganischen Basen lassen sich derartige tertiäre Amine gewünschtenfalls leicht durch Destillation aus dem Reaktionsgemisch entfernen; gegebenenfalls können sie auch in der Formose verbleiben und wirken dann beispielsweise bei der Weiterverwendung der Formose als Ausgangskomponente für die Herstellung von Polyurethankunststoffen als Katalysatoren für die Polyisocyanat-Polyadditionsreaktion. Wie weiter gefunden wurde, ist die pH-Führung während der Formaldehydkondensation bei Verwendung von tertiären Aminen als Base überraschenderweise weitgehend unkritisch: der pH-Wert während der Formosebildung kann in relativ weiten Grenzen schwanken; er liegt im allgemeinen zwischen 4,5 und 9, vorzugsweise zwischen 5 und 7.

Gegenstand der vorliegenden Erfindung ist somit ein Verfahren zur Herstellung von niedermolekularen Polyhydroxylverbindungen durch Selbstkondensation von Formaldehydhydrat in Gegenwart von löslichen oder unlöslichen Verbindungen von Metallen der zweiten bis vierten Hauptgruppe oder der zweiten bis achten Nebengruppe des periodischen Systems

<u>Le A 18 370</u>

der Elemente als Katalysator sowie gegebenenfalls in Gegenwart von zur Endiolbildung befähigten Verbindungen als Cokatalysator und/oder von niedermolekularen und/oder höhermolekularen Polyhydroxylverbindungen, welches dadurch gekennzeichnet ist, daß der pH-Wert während der Kondensationsreaktion durch die Zugabe von 5 bis 200 Milliäquivalenten, vorzugsweise 10 bis 70 Milliäquivalenten, eines tertiären Amins, bezogen auf 1 Mol Formaldehyd, gesteuert wird.

Überraschenderweise tritt der erfindungsbegründende Effekt der weitgehenden Zurückdrängung der gekreuzten Cannizzaro-Reaktion nur bei der Verwendung teritärer Amine auf, während sich primäre oder sekundäre Amine ähnlich wie anorganische Basen verhalten, also zu einer merklichen gekreuzten Cannizzaro-Reaktion Anlaß geben (s. die Vergleichsversuche im Beispielteil).

Erfindungsgemäß bevorzugt sind solche tertiären Amine, welche in ß-Stellung zum tertiären Stickstoffatom ein Elektronen anziehendes Heteroatom enthalten, beispielsweise ein weiteres tertiäres Stickstoffatom, eine Thioäthergruppe oder besonders bevorzugt eine Äthergruppe. Beispiele für erfindungsgemäß zu verwendende tertiäre Amine sind Trialkylamine wie Triäthylamin, Tri-n-propylamin, Tri-n-butylamin, N,N-Dialkylbenzylamine mit 1 bis 5, vorzugweise 1 oder 2, C-Atomen in den Alkylgruppen, N-Alkylpiperidine, N,N'-Dialkylpiperazine und N-Alkylmorpholine mit jeweils 1 bis 5, vorzugsweise 1 oder 2, C-Atomen in den Alkylgruppen, N-Phenylmorpholin, N-Benzylmorpholin, 1,2-Bis-morpholyl-äthan sowie Anlagerungsprodukte von Äthylenoxid und/oder Propylenoxid an Morpholin oder Piperazin mit einem Molekulargewicht bis etwa 2000, vorzugsweise bis ca.

Le A 18 370

1000. Diese letztgenannten Verbindungen sind ebenso wie andere, für die Polyurethanherstellung an sich bekannte, basische Polyäther (Alkoxylierungsprodukte von Ammoniak, primären und sekundären Mono- oder Polyaminen) erfindungsgemäß von besonderem Interesse, weil sie in der Formose verbleiben können und später bei der Verwendung der Formose als Ausgangskomponente zur Herstellung von Polyurethankunststoffen einbaubare Katalysatoren für die Polyisocyanat-Polyadditionsreaktion darstellen.

Die Selbstkondensation des Formaldehydhydrats unter Bildung von Hydroxyaldehyden und Hydroxyketonen wird erfindungsgemäß durch unlösliche, vorzugsweise jedoch in Wasser lösliche Verbindungen (insbesondere Salze) von Metallen der zweiten bis vierten Hauptgruppe oder der zweiten bis achten Nebengruppe des periodischen Systems der Elemente katalysiert. Von besonderem technischem Interesse als Katalysatoren sind auch mit Ionen dieser Metalle beladene Ionenaustauscher. Im allgemeinen werden erfindungsgemäß ca. 0,01 bis 10 Gew.-%, vorzugsweise 0,05 bis 5 Gew.-%, besonders bevorzugt 0,1 bis 1 Gew.-%, bezogen auf eingesetzten Formaldehyd, des Katalysators mitverwendet. Die Verwendung von stark basischen Oxiden oder Hydroxiden dieser Metalle (insbesondere von Erdalkalihydroxiden) ist erfindungsgemäß weniger bevorzugt, da durch sie die gekreuzte Cannizzaro-Reaktion wieder begünstigt wird. Sollen Metallhydroxide als Katalysator eingesetzt werden, dann empfiehlt es sich daher, möglichst geringe Mengen des Katalysators einzusetzen, also an der unteren Grenze des obengenannten Bereiches zu arbeiten. Erfindungsgemäß bevorzugt werden Formiate, Acetate, Phenolate, Thiophenolate, Salicylate, Carbonate und Nitrate

Le A 18 370

der Metalle bzw. die bereits erwähnten mit Metallionen beladenen Ionenaustauscher als Katalysator verwendet. Erfindungsgemäß besonders bevorzugte Katalysatoren sind Verbindungen des zweiwertigen Bleis, insbesondere Bleiacetat.

Vorzugsweise läßt man erfindungsgemäß die Selbstkondensation des Formaldehydhydrats in Gegenwart von zur Endiolbildung befähigten Verbindungen als Co-Katalysator ablaufen. Im allgemeinen werden dabei 0,1 bis 50, vorzugsweise 1 bis 20, besonders bevorzugt 5 bis 15, Gew.-%, bezogen auf Formaldehyd, an zur Endiolbildung befähigten Verbindungen eingesetzt. Prinzipiell kommen für diesen Zweck beliebige Verbindungen in Frage, welche in $\alpha$-Stellung zu einer Carbonylgruppe eine Hydroxylgruppe enthalten; erfindungsgemäß bevorzugte Co-Katalysatoren sind jedoch Formose selbst und Oxidationsprodukte mehrwertiger Alkohole (s. in diesem Zusammenhang die eingangs zitierte Literatur).

Anstelle dieser zur Endiolbildung befähigten Verbindungen bzw. zusätzlich dazu können im erfindungsgemäßen Verfahren auch Polyhydroxylverbindungen in einer Menge bis zu 200 Gew.-%, vorzugsweise 10 bis 100 Gew.-%, bezogen auf eingesetzten Formaldehyd, mitverwendet werden. Es kommen in diesem Zusammenhang die in der deutschen Patentanmeldung P 2 714 104.4 beschriebenen Polyhydroxylverbindungen bzw. die weiter unten als Ausgangskomponente bei der Herstellung von Polyurethankunststoffen genannten Polyole in Betracht.

Wie schon erwähnt, ist die Verfahrensführung bei der erfindungsgemäßen Kondensation von Formaldehydhydrat in Gegenwart von tertiären Aminen weitgehend unkritisch. Im

Le A 18 370

allgemeinen wird bei Temperaturen von 10 bis 150°C, vorzugsweise 70 bis 110°C, besonders bevorzugt 80 bis 100°C, und in einem pH-Bereich von vorzugsweise 4,5 bis 9, besonders bevorzugt 5 bis 7, gearbeitet. Im allgemeinen setzt man erfindungsgemäß 10 bis 70 Gew.-%, vorzugsweise 20 bis 65 Gew.-%, besonders bevorzugt 30 bis 50 Gew.-%, Formaldehyd enthaltende wäßrige und/oder alkoholische Formalinlösungen und/oder Paraformaldehyddispersionen ein. Je nach dem Säuregehalt dieser Ausgangskomponenten richtet sich die erfindungsgemäß zuzusetzende Menge des tertiären Amins. Sie liegt jedoch im allgemeinen zwischen 5 und 200, vorzugsweise zwischen 10 und 70, Milliäquivalenten an tertiären Stickstoffatomen pro Mol Formaldehyd. Man kann erfindungsgemäß die Gesamtmenge der organischen Base gleich am Beginn der Kondensationsreaktion zufügen, da infolge der weitgehenden Unterdrückung von gekreuzten Cannizzaro-Reaktionen während der Formosebildung nur sehr wenig Säuren entstehen, so daß der pH-Wert während der Kondensationsreaktion nur sehr langsam abnimmt. Bei längeren Reaktionszeiten (falls ein möglichst vollständiger Umsatz des Formaldehyds gewünscht ist) treten jedoch in geringem Umfang doch gekreuzte Cannizzaro-Reaktionen auf, so daß es dann zweckmäßig ist, die organische Base portionsweise oder kontinuierlich zuzufügen. Durch die Art der Zugabe des tertiären Amins hat man es auch in der Hand, die Reaktionsgeschwindigkeit in der gewünschten Weise zu beeinflussen.

Selbstverständlich läßt sich das erfindungsgemäße Verfahren auch kontinuierlich, beispielsweise in einer Rührkesselkaskade, ausführen. Durch Variation der Verweilzeit und des pH-Wertes in den einzelnen Rührkesseln läßt sich

0000910

bei dieser Verfahrensvariante der Restformaldehydgehalt exakt einstellen. Die Produktverteilung in der so erhaltenen Formose ist auf diese Weise leicht in weiten Grenzen variierbar und reproduzierbar. Auf ähnlich günstige Weise gelingt die Herstellung von Formose nach dem erfindungsgemäßen Verfahren auch in einem kontinuierlich betriebenen Reaktionsrohr, in welches zur Aufrechterhaltung des gewünschten pH-Wertes im gesamten Reaktionsvolumen an einer oder mehreren Stellen des Rohres kontinuierlich die organische Base in der notwendigen Menge hinzugefügt werden kann. Auch in diesem Fall ist es möglich, durch Variation der Durchflußzeiten und der pH-Führung die Produktverteilung der Formose in weiten Grenzen zu verändern.

Die erfindungsgemäß erhaltenen Formosen sind wertvolle Ausgangsmaterialien für eine Vielzahl anwendungstechnisch interessanter Produkte; insbesondere sind sie als Polyolkomponente bei der Herstellung von Polyurethankunststoffen geeignet.

Gegenstand der Erfindung ist somit auch ein Verfahren zur Herstellung von gegebenenfalls zellförmigen Polyurethankunststoffen durch Umsetzung von

A) Polyisocyanaten mit

B) niedermolekularen Polyhydroxylverbindungen sowie gegebenenfalls

C) höhermolekularen Polyhydroxylverbindungen, weiteren Kettenverlängerungsmitteln, Treibmitteln, Katalysatoren und weiteren an sich bekannten Zusatzstoffen,

**Le A 18 370**

welches dadurch gekennzeichnet ist, daß als Komponente B
die erfindungsgemäß erhaltenen Formosen eingesetzt werden.

Als Polyisocyanate kommen in diesem Zusammenhang beispielsweise die aliphatischen, cycloaliphatischen, araliphatischen,
aromatischen und heterocyclischen Polyisocyanate in Frage,
wie sie z.B. von W. Siefken in Justus Liebigs Annalen der
Chemie, 562, Seiten 75 bis 136, beschrieben werden, beispielsweise Äthylen-diisocyanat, 1,4-Tetramethylendiisocyanat, 1,6-Hexamethylendiisocyanat, 1,12-Dodecandiisocyanat,
Cyclobutan-1,3-diisocyanat, Cyclohexan-1,3- und -1,4-Diisocyanat sowie beliebige Gemische dieser Isomeren, 1-Isocya-
nato-3,3,5-trimethyl-5-isocyanatomethyl-cyclohexan (DAS
1 202 785, amerikanische Patentschrift 3 401 190), 2,4-
und 2,6-Hexahydrotoluylendiisocyanat sowie beliebige Gemische dieser Isomeren, Hexahydro-1,3- und/oder -1,4-phenylen-diisocyanat, Perhydro-2,4'- und/oder -4,4'-diphenylmethan-diisocyanat, 1,3- und 1,4-Phenylendiisocyanat, 2,4-
und 2,6-Toluylendiisocyanat sowie beliebige Gemische dieser Isomeren, Diphenylmethan-2,4'- und/oder -4,4'-diisocyanat, Naphthylen-1,5-diisocyanat, Triphenylmethan-4,4'-4"-
triisocyanat, Polyphenyl-polymethylen-polyisocyanate, wie
sie durch Anilin-Formaldehyd-Kondensation und anschließende
Phosgenierung erhalten und z.B. in den britischen Patentschriften 874 430 und 848 671 beschrieben werden, m- und p-
Isocyanatophenylsulfonyl-isocyanate gemäß der amerikanischen Patentschrift 3 454 606, perchlorierte Arylpolyisocyanate, wie sie z.B. in der deutschen Auslegeschrift
1 157 601 (amerikanische Patentschrift 3 277 138) beschrieben werden, Carbodiimidgruppen aufweisende Polyisocyanate,

**Le A 18 370**

wie sie in der deutschen Patentschrift 1 092 007 (amerikanische Patentschrift 3 152 162) beschrieben werden, Diisocyanate, wie sie in der amerikanischen Patentschrift 3 492 330 beschrieben werden, Allophanatgruppen aufweisende Polyisocyanate, wie sie z.B. in der britischen Patentschrift 994 890, der belgischen Patentschrift 761 626 und der veröffentlichten holländischen Patentanmeldung 7 102 524 beschrieben werden, Isocyanuratgruppen aufweisende Polyisocyanate, wie sie z.B. in der amerikanischen Patentschrift 3 001 973, in den deutschen Patentschriften 1 022 789, 1 222 067 und 1 027 394 sowie in den deutschen Offenlegungsschriften 1 929 034 und 2 004 048 beschrieben werden, Urethangruppen aufweisende Polyisocyanate, wie sie z.B. in der belgischen Patentschrift 752 261 oder in der amerikanischen Patentschrift 3 394 164 beschrieben werden, acylierte Harnstoffgruppen aufweisende Polyisocyanate gemäß der deutschen Patentschrift 1 230 778, Biuretgruppen aufweisende Polyisocyanate, wie sie z.B. in der deutschen Patentschrift 1 101 394 (amerikanische Patentschriften 3 124 605 und 3 201 372) sowie in der britischen Patentschrift 889 050 beschrieben werden, durch Telomerisationsreaktionen hergestellte Polyisocyanate, wie sie z.B. in der amerikanischen Patentschrift 3 654 106 beschrieben werden, Estergruppen aufweisende Polyisocyanate, wie sie z.B. in den britischen Patentschriften 965 474 und 1 072 956, in der amerikanischen Patentschrift 3 567 763 und in der deutschen Patentschrift 1 231 688 genannt werden, Umsetzungsprodukte der obengenannten Isocyanate mit Acetalen gemäß der deutschen Patentschrift 1 072 385 und polymere Fettsäurereste enthaltende Polyisocyanate gemäß der amerikanischen Patentschrift 3 455 883.

Le A 18 370

Es ist auch möglich, die bei der technischen Isocyanatherstellung anfallenden, Isocyanatgruppen aufweisenden Destillationsrückstände, gegebenenfalls gelöst in einem oder mehreren
der vorgenannten Polyisocyanate, einzusetzen. Ferner ist es
möglich, beliebige Mischungen der vorgenannten Polyisocyanate
zu verwenden.

Besonders bevorzugt werden in der Regel die technisch leicht
zugänglichen Polyisocyanate, z.B. das 2,4- und 2,6-Toluylendiisocyanat sowie beliebige Gemische dieser Isomeren ("TDI"),
Polyphenyl-polymethylen-polyisocyanate, wie sie durch Anilin-
Formaldehyd-Kondensation und anschließende Phosgenierung hergestellt werden ("rohes MDI") und Carbodiimidgruppen, Urethangruppen, Allophanatgruppen, Isocyanuratgruppen, Harnstoffgruppen oder Biuretgruppen aufweisenden Polyisocyanate ("modifizierte Polyisocyanate").

Geeignete höhermolekulare Polyhydroxylverbindungen, speziell solche vom Molekulargewicht 800 bis 10 000, vorzugsweise 1000
bis 6000, sind z.B. mindestens zwei, in der Regel 2
bis 8, vorzugsweise aber 2 bis 4, Hydroxylgruppen aufweisende Polyester, Polyäther, Polythioäther, Polyacetale,
Polycarbonate und Polyesteramide, wie sie für die Herstellung von homogenen und von zellförmigen Polyurethanen
an sich bekannt sind.

Die in Frage kommenden Hydroxylgruppen aufweisenden Polyester sind z.B. Umsetzungsprodukte von mehrwertigen, vorzugsweise zweiwertigen und gegebenenfalls zusätzlich dreiwertigen Alkoholen mit mehrwertigen, vorzugsweise zweiwertigen, Carbonsäuren. Anstelle der freien Polycarbonsäuren können auch die entsprechenden Polycarbonsäureanhydride oder entsprechende Polycarbonsäureester von
niedrigen Alkoholen oder deren Gemische zur Herstellung
der Polyester verwendet werden. Die Polycarbonsäuren
können aliphatischer, cycloaliphatischer, aromatischer
und/oder heterocyclischer Natur sein und gegebenenfalls,
z.B. durch Halogenatome, substituiert und/oder ungesättigt
sein.

Le A 18 370

Als Beispiele hierfür seien genannt: Bernsteinsäure, Adipinsäure, Korksäure, Azelainsäure, Sebacinsäure, Phthalsäure, Isophthalsäure, Trimellitsäure, Phthalsäureanhydrid, Tetrahydrophthalsäureanhydrid, Hexahydrophthalsäureanhydrid, Tetrachlorphthalsäureanhydrid, Endomethylentetrahydrophthalsäureanhydrid, Glutarsäureanhydrid, Maleinsäure, Maleinsäureanhydrid, Fumarsäure, dimere und trimere Fettsäuren wie Ölsäure, gegebenenfalls in Mischung mit monomeren Fettsäuren, Terephthalsäuredimethylester und Terephthalsäure-bis-glykolester.

Als mehrwertige Alkohole kommen z.B. Äthylenglykol, Propylenglykol-(1,2) und -(1,3), Butylenglykol-(1,4) und -(2,3), Hexandiol-(1,6), Octandiol-(1,8), Neopentylglykol, Cyclohexandimethanol(1,4-Bis-hydroxymethylcyclohexan), 2-Methyl-1,3-propandiol, Glycerin, Trimethylolpropan, Hexantriol-(1,2,6), Butantriol-(1,2,4), Trimethyloläthan, Pentaerythrit, Chinit, Mannit und Sorbit, Methylglykosid, ferner Diäthylenglykol, Triäthylenglykol, Tetraäthylenglykol, Polyäthylenglykole, Dipropylenglykol, Polypropylenglykole, Dibutylenglykol und Polybutylenglykole in Frage. Die Polyester können anteilig endständige Carboxylgruppen aufweisen. Auch Polyester aus Lactonen, z.B. ε-Caprolacton oder Hydroxycarbonsäuren, z.B. ω-Hydroxycapronsäure, sind einsetzbar.

Auch die erfindungsgemäß in Frage kommenden, mindestens zwei, in der Regel zwei bis acht, vorzugsweise zwei bis drei, Hydroxylgruppen aufweisenden Polyäther sind solche der an sich bekannten Art und werden z.B. durch Poly-

Le A 18 370

0000910

merisation von Epoxiden wie Äthylenoxid, Propylenoxid, Butylenoxid, Tetrahydrofuran, Styroloxid oder Epichlorhydrin mit sich selbst, z.B. in Gegenwart von $BF_3$, oder durch Anlagerung dieser Epoxide, gegebenenfalls im Gemisch oder nacheinander, an Startkomponenten mit reaktionsfähigen Wasserstoffatomen wie Wasser, Alkohole, Ammoniak oder Amine, z.B. Äthylenglykol, Propylenglykol-(1,3) oder -(1,2), Trimethylolpropan, 4,4'-Dihydroxy-diphenylpropan, Anilin, Äthanolamin oder Äthylendiamin hergestellt. Auch Sucrosepolyäther, wie sie z.B. in den deutschen Auslegeschriften 1 176 358 und 1 064 938 beschrieben werden, kommen erfindungsgemäß in Frage. Vielfach sind solche Polyäther bevorzugt, die überwiegend (bis zu 90 Gew.-%, bezogen auf alle vorhandenen OH-Gruppen im Polyäther) primäre OH-Gruppen aufweisen. Auch durch Vinylpolymerisate modifizierte Polyäther, wie sie z.B. durch Polymerisation von Styrol und Acrylnitril in Gegenwart von Polyäthern entstehen (amerikanische Patentschriften 3 383 351, 3 304 273, 3 523 093, 3 110 695, deutsche Patentschrift 1 152 536), sind geeignet, ebenso OH-Gruppen aufweisende Polybutadiene.

Unter den Polythioäthern seien insbesondere die Kondensationsprodukte von Thiodiglykol mit sich selbst und/ oder mit anderen Glykolen, Dicarbonsäuren, Formaldehyd, Aminocarbonsäuren oder Aminoalkoholen angeführt. Je nach den Co-Komponenten handelt es sich bei den Produkten um Polythiomischäther, Polythioätherester oder Polythioätheresteramide.

Le A 18 370

Als Polyacetale kommen z.B. die aus Glykolen, wie Diäthylenglykol, Triäthylenglykol, 4,4'-Dioxäthoxydiphenyldimethylmethan, Hexandiol und Formaldehyd herstellbaren Verbindungen in Frage. Auch durch Polymerisation cyclischer Acetale lassen sich erfindungsgemäß geeignete Polyacetale herstellen.

Als Hydroxylgruppen aufweisende Polycarbonate kommen solche der an sich bekannten Art in Betracht, die z.B. durch Umsetzung von Diolen wie Propandiol-(1,3), Butandiol-(1,4) und/oder Hexandiol-(1,6), Diäthylenglykol, Triäthylenglykol oder Tetraäthylenglykol mit Diarylcarbonaten, z.B. Diphenylcarbonat, oder Phosgen hergestellt werden können.

Zu den Polyesteramiden und Polyamiden zählen z.B. die aus mehrwertigen gesättigten und ungesättigten Carbonsäuren bzw. deren Anhydriden und mehrwertigen gesättigten und ungesättigten Aminoalkoholen, Diaminen, Polyaminen und ihren Mischungen gewonnenen, vorwiegend linearen Kondensate.

Auch bereits Urethan- oder Harnstoffgruppen enthaltende Polyhydroxylverbindungen sowie gegebenenfalls modifizierte natürliche Polyole, wie Rizinusöl, Kohlenhydrate oder Stärke, sind verwendbar. Auch Anlagerungsprodukte von Alkylenoxiden an Phenol-Formaldehyd-Harze oder auch an Harnstoff-Formaldehydharze sind erfindungsgemäß einsetzbar.

Le A 18 370

- 19 -                                    0000910

Vertreter dieser erfindungsgemäß gegebenenfalls mit zuverwendende Verbindungen sind z.B. in High Polymers, Vol. XVI, "Polyurethanes, Chemistry and Technology", verfaßt von Saunders-Frisch, Interscience Publishers, New York, London, Band I, 1962, Seiten 32-42 und Seiten 44-54 und Band II, 1964, Seiten 5-6 und 198-199, sowie im Kunststoff-Handbuch, Band VII, Vieweg-Höchtlen, Carl-Hanser-Verlag, München, 1966, z.B. auf den Seiten 45-71, beschrieben.

Selbstverständlich können Mischungen der obengenannten Verbindungen mit mindestens zwei gegenüber Isocyanaten reaktionsfähigen Wasserstoffatomen mit einem Molekulargewicht von 800 - 10 000, z.B. Mischungen von Polyäthern und Polyestern, eingesetzt werden.

Als erfindungsgemäß gegebenenfalls einzusetzende Ausgangskomponenten kommen auch Verbindungen mit mindestens zwei gegenüber Isocyanaten reaktionsfähigen Wasserstoffatomen von einem Molekulargewicht 32-400 in Frage. Auch in diesem Fall versteht man hierunter Hydroxylgruppen und/oder Aminogruppen und/oder Thiolgruppen und/oder Carboxylgruppen aufweisende Verbindungen, vorzugsweise Hydroxylgruppen und/oder Aminogruppen aufweisende Verbindungen, die als Kettenverlängerungsmittel oder Vernetzungsmittel dienen. Diese Verbindungen weisen in der Regel 2 bis 8 gegenüber Isocyanaten reaktionsfähige Wasserstoffatome auf, vorzugsweise 2 oder 3 reaktionsfähige Wasserstoffatome.

Le A 18 370

Als Beispiele für derartige Verbindungen seien genannt: Äthylenglykol, Propylenglykol-(1,2) und -(1,3), Butylenglykol-(1,4) und -(2,3), Pentandiol-(1,5), Hexandiol-(1,6), Octandiol-(1,8), Neopentylglykol, 1,4-Bishydroxymethyl-cyclohexan, 2-Methyl-1,3-propandiol, Glyzerin, Trimethylolpropan, Hexantriol-(1,2,6), Trimethyloläthan, Pentaerythrit, Chinit, Mannit und Sorbit, Diäthylenglykol, Triäthylenglykol, Tetraäthylenglykol, Polyäthylenglykole mit einem Molekulargewicht bis 400, Dipropylenglykol, Polypropylenglykole mit einem Molekulargewicht bis 400, Dibutylenglykol, Polybutylenglykole mit einem Molekulargewicht bis 400, 4,4'-Dihydroxy-diphenylpropan, Di-hydroxymethyl-hydrochinon, Äthanolamin, Diäthanolamin, Triäthanolamin, 3-Aminopropanol, Äthylendiamin, 1,3-Diaminopropan, 1-Mercapto-3-aminopropan, 4-Hydroxy- oder -Amino-phthalsäure, Bernsteinsäure, Adipinsäure, Hydrazin, N,N'-Dimethylhydrazin, 4,4'-Diaminodiphenylmethan, Toluylendiamin, Methylen-bis-chloranilin, Methylen-bis-anthranilsäureester Diaminobenzoesäureester und die isomeren Chlorphenylendiamine.

Auch in diesem Fall können Mischungen von verschiedenen Verbindungen mit mindestens zwei gegenüber Isocyanaten reaktionsfähigen Wasserstoffatomen mit einem Molekulargewicht von 32-400 verwendet werden.

Le A 18 370

Erfindungsgemäß können jedoch auch Polyhydroxylverbindungen eingesetzt werden, in welchen hochmolekulare Polyaddukte bzw. Polykondensate in feindisperser oder gelöster Form enthalten sind. Derartige modifizierte Polyhydroxylverbindungen werden erhalten, wenn man Polyadditionsreaktionen (z.B. Umsetzungen zwischen Polyisocyanaten und aminofunktionellen Verbindungen) bzw. Polykondensationsreaktionen (z.B. zwischen Formaldehyd und Phenolen und/oder Aminen) direkt in situ in den oben genannten, Hydroxylgruppen aufweisenden Verbindungen ablaufen läßt. Derartige Verfahren sind beispielsweise in den Deutschen Auslegeschriften 1 168 075 und 1 260 142, sowie den Deutschen Offenlegungsschriften 2 324 134, 2 423 984, 2 512 385, 2 513 815, 2 550 796, 2 550 797, 2 550 833 und 2 550 862 beschrieben. Es ist aber auch möglich, gemäß US-Patent 3 869 413 bzw. Deutscher Offenlegungsschrift 2 550 860 eine fertige wäßrige Polymerdispersion mit einer Polyhydroxylverbindung zu vermischen und anschließend aus dem Gemisch das Wasser zu entfernen.

Bei der Verwendung von modifizierten Polyhydroxylverbindungen der oben genannten Art als Ausgangskomponente im Polyisocyanat-Polyadditionsverfahren entstehen in vielen Fällen Polyurethankunststoffe mit wesentlich verbesserten mechanischen Eigenschaften.

Le A 18 370

0000910

Die ausschließliche Umsetzung der erfindungsgemäß zugänglichen Polyhydroxylverbindungen (ohne Mitverwendung anderer gegenüber Isocyanaten reaktiver Komponenten) mit stark elastifizierenden Polyisocyanaten,wie z.B. Polyisocyanaten mit Biuretstruktur (DAS 1 543 178) führt zu harten, lichtechten, kratz- und lösungsmittelfesten Beschichtungen und Lacken.

Durch Propoxylierung oder/und Oxäthylierung der Formose lassen sich ferner Polyätheralkohole hoher Funktionalität gewinnen, die in hohen OH-Zahl-Bereichen für die Herstellung von harten bzw. halbharten zellförmigen Polyurethankunststoffen und bei niedrigen OH-Zahlen als Ausgangsmaterialien für hochelastische Polyurethanschaumstoffe Verwendung finden.

Durch Umsetzung der erfindungsgemäß hergestellten Formose mit mehrwertigen Carbonsäuren der obengenannten Art, z.B. Phthalsäure, Isophthalsäure, Terephthalsäure, Tetra- und Hexahydrophthalsäure, Adipinsäure oder Maleinsäure nach den üblichen Verfahren der Polyesterkondensation, wie sie beispielsweise in Houben-Weyl, Methoden der organischen Chemie, Bd. XIV 12, S. 40 beschrieben sind, lassen sich stark verzweigte Polyester synthetisieren, die als Zusätze zu Alkydharzen deren Härte verbessern. Die Hydroxylgruppen enthaltende Polyester, die aus den erfindungsgemäß hergestellten Hydroxylverbindungen synthetisiert werden,sind selbstverständlich ebenfalls als Ausgangskomponente zur Herstellung von Polyurethankunststoffen brauchbar.

Le A 18 370

Die erfindungsgemäß hergestellten Formosen lassen sich auch sehr leicht mit langkettigen, aliphatischen Monocarbonsäuren, wie Capryl-, Caprin-, Laurin-, Myristin-, Palmitin-, Stearin-, Öl-, Linol-, Arachidon-, oder Behensäure, sowie deren Derivaten, wie z.B. den Methyl- oder Äthylestern oder auch den Anhydriden bzw. gemischten Anhydriden zu hydroxylgruppenhaltigen Estern umsetzen. Diese stellen ebenso wie Oxäthylierungsprodukte der Polyole oder auch Umsetzungsprodukte der erfindungsgemäß zugänglichen Polyhydroxylverbindungen mit langkettigen Monoisocyanaten, wie n-Octyl-, n-Decyl-, n-Dodecyl-, Myristyl-, Cetyl- oder Stearylisocyanat zu Carbamidsäureestern(siehe z.B. K. Lindner, Tenside Bd. III, Wissenschaftliche Verlagsgesellschaft Stuttgart, 1964, S. 2336) nichtionogene, oberflächenaktive Verbindungen dar, die als wertvolle Emulgatoren, Netzmittel oder Weichmacher Verwendung finden können.

Die erfindungsgemäßen Formosen lassen sich auch als Feuchthaltemittel in Kosmetika und Kunststoffen verwenden. Sie können aber z.B. auch als Gefrierschutzmittel dienen.

Ebenso ist ihr Einsatz als kohlenhydrathaltiges Substrat in Nährböden von Mikroorganismen möglich. Hierzu haben sich besonders diejenigen Verfahrensprodukte bewährt, die hauptsächlich aus 5 und 6 Kohlenstoffatome enthaltenden Hydroxyaldehyden und Hydroxyketonen bestehen.

Die folgenden Beispiele erläutern das erfindungsgemäße Verfahren. Wenn nicht anders vermerkt, sind Zahlenwerte als Gewichtsteile bzw. Gewichtsprozente zu verstehen.

Le A 18 370

Beispiel 1

1000 g (12,3 Mol Formaldehyd) einer 37 %igen wäßrigen Formalinlösung werden zusammen mit 10 g (0,27 Mol) Blei(II)acetat
und 50 g der unten beschriebenen Formose als Co-Katalysator
auf Rückflußtemperatur erhitzt. Dann wird das Heizbad entfernt und langsam Dimethylbenzylamin zudosiert, so daß die
Mischung von selbst weitersiedet. Nach 55 Minuten ist der
Formaldehydrestgehalt auf 0,3 % gefallen. Man beendet die
Reaktion durch Kühlen.

Verbrauch an Dimethylbenzylamin: 20 g.
Nach Einengen am Wasserstrahlvakuum erhält man 425 g eines
2,4 % Wasser enthaltenden Gemisches mehrwertiger Alkohole,
Hydroxyaldehyde und Hydroxyketone mit einem Gehalt an reduzierenden Anteilen, berechnet als Glucose, von 71,2 %, einer
Viskosität bei 50$^{o}$C von 1810 mPa.s und einer OH-Zahl von
760. Diese Mischung kann nach bekannter Verfahrensweise
mit Raney-Nickel als Katalysator hydriert werden. Man erhält ein Gemisch mehrwertiger Alkohole mit einem Wassergehalt von 3,8 % und einer Viskosität bei 50$^{o}$C von 616 mPa.s.

Herstellung des Co-Katalysators:

3000 Teile einer 37 %igen wäßrigen Formaldehydlösung
(37 Mole Formaldehyd) werden auf 70 -90$^{o}$C erhitzt. Bei
dieser Temperatur werden 30 Teile (0,08 Mol) Blei(II)-
acetat zugegeben. Die Mischung wird dann weiter auf 100$^{o}$C
erhitzt und bei dieser Temperatur durch Zutropfen einer
15 %igen Ca(OH)$_2$-Suspension auf einen pH-Wert von 6,7
eingestellt.

Le A 18 370

Nach 6 Stunden ist der Formaldehydgehalt auf einen Wert von 20 % abgesunken und die Ca(OH)$_2$-Zufuhr wird gestoppt. Der pH-Wert der Reaktionsmischung fällt nun langsam ab. Nachdem ein pH-Wert von 5,7 erreicht ist, wird die Mischung durch Zugabe weiterer Ca(OH)$_2$-Suspension auf diesem Wert gehalten. Nach weiteren 7,5 Stunden ist ein Restformaldehydgehalt von 0,5 % erreicht und die Reaktionsmischung wird gekühlt. Man erhält eine ca. 37 %ige Lösung eines Co-Katalysator-Gemisches, bestehend aus Hydroxyaldehyden und Hydroxyketonen, bei dem das Molverhältnis der Verbindungen mit 3 C-Atomen und der Verbindungen mit 4 C-Atomen = 0,75, das Molverhältnis der Verbindungen mit 4 C-Atomen und der Verbindungen mit 5 C-Atomen 0,23 und das Molverhältnis der Verbindungen mit 5 C-Atomen und der Verbindungen mit 6 C-Atomen 0,67 beträgt.

Beispiel 2

1000 g (12,3 Mol Formaldehyd) einer 37 %igen wäßrigen Formalinlösung werden zusammen mit 10 g (0,27 Mol) Blei(II)acetat und 50 g des Co-Katalysators aus Beispiel 1 auf Rückflußtemperatur erhitzt. Dann wird das Heizbad entfernt und langsam Methylpiperidin zudosiert, so daß die Mischung von selbst weitersiedet. Nach 50 Minuten ist der Formaldehydrestgehalt auf 1,5 % gefallen. Man beendet die Reaktion durch Kühlen.
Verbrauch an Methylpiperidin: 20 g.
Nach Einengen am Wasserstrahlvakuum erhält man 321 g eines 3,3 % Wasser enthaltenden Gemisches mehrwertiger Alkohole,

Le A 18 370

Hydroxyaldehyde und Hydroxyketone mit einem Gehalt an reduzierenden Anteilen, berechnet als Glucose, von 70,2 %, einer Viskosität bei 50°C von 24 244 mPa.s und einer OH-Zahl von 846.

**Beispiel 3**

1000 g (12,3 Mol Formaldehyd) einer 37 %igen wäßrigen Formalinlösung werden zusammen mit 10 g (0,27 Mol) Blei(II)acetat und 50 g des Co-Katalysators aus Beispiel 1 auf Rückflußtemperatur erhitzt. Dann wird das Heizbad entfernt und langsam Methylmorpholin zudosiert, so daß die Mischung von selbst weitersiedet. Nach 20 Minuten ist kein Formaldehyd mehr nachweisbar. Man kühlt ab.
Verbrauch an Methylmorpholin: 26 g.
Nach Einengen am Wasserstrahlvakuum erhält man 373 g eines 1,8 % Wasser enthaltenden Gemisches mehrwertiger Alkohole, Hydroxyaldehyde und Hydroxyketone mit einem Gehalt an reduzierenden Anteilen, berechnet als Glucose, von 80,4 %, einer Viskosität bei 50°C von 58 190 mPa.s und einer OH-Zahl von 898.

**Beispiel 4**   (Vergleich)

1000 g (12,3 Mol Formaldehyd) einer 37 %igen wäßrigen Formalinlösung werden zusammen mit 10 g (0,27 Mol) Blei(II)-acetat und 50 g des Co-Katalysators aus Beispiel 1 auf Rückflußtemperatur erhitzt. Dann wird das Heizbad entfernt und langsam Piperidin zudosiert, so daß die Mischung von selbst weitersiedet. Nach 85 Minuten ist der Formaldehydrestgehalt auf 1,8 % gefallen. Man beendet die Reaktion durch Kühlen.

Le A 18 370

Verbrauch an Piperidin: 58 g.

Nach Einengen am Wasserstrahlvakuum erhält man 401 g eines 1,7 % Wasser enthaltenden Gemisches mehrwertiger Alkohole, Hydroxyaldehyde und Hydroxyketone mit einem Gehalt an reduzierenden Anteilen, berechnet als Glucose, von 47,7 %, einer Viskosität bei 50 °C von 21565 mPa.s und einer OH-Zahl von 668.

Das Beispiel zeigt, daß bei Verwendung eines sekundären Amins längere Reaktionszeiten für die Formaldehydkondensation erforderlich sind, sowie daß der Basenverbrauch höher liegt und die Ausbeute an reduzierenden Zuckern schlechter ist, was beides beweist, daß in erheblichem Umfang Cannizzaro-Reaktionen eingetreten sind.

Beispiel 5    (Vergleich)

1000 g (12,3 Mol Formaldehyd) einer 37 %igen wäßrigen Formalinlösung werden zusammen mit 10 g (0,27 Mol) Blei(II)acetat und 50 g des Co-Katalysators aus Beispiel 1 auf Rückflußtemperatur erhitzt. Dann wird das Heizbad entfernt und langsam Morpholin zudosiert, so daß die Mischung von selbst weitersiedet. Nach 45 Minuten ist der Formaldehydrestgehalt auf 2,7 % gefallen. Man beendet die Reaktion durch Kühlen. Verbrauch an Morpholin: 60 g.

Nach Einengen am Wasserstrahlvakuum erhält man 382 g eines 3,7 % Wasser enthaltenden Gemisches mehrwertiger Alkohole, Hydroxyaldehyde und Hydroxyketone mit einem Gehalt an reduzierenden Anteilen, berechnet als Glucose, von 48,8 %, einer Viskosität bei 50°C von 1379 mPa.s und einer OH-Zahl von 611.

Le A 18 370

Beispiel 6   (Vergleich)

1000 g (12,3 Mol Formaldehyd) einer 37 %igen wäßrigen Formalinlösung werden zusammen mit 10 g (0,27 Mol) Blei(II)acetat und
50 Teilen des Co-Katalysators aus Beispiel 1 auf Rückflußtemperatur erhitzt. Dann wird das Heizbad entfernt und langsam 2-(2-Aminoäthylamino)äthanol-1 zudosiert, so daß die
Mischung von selbst weitersiedet. Nach 55 Minuten bei einem
pH-Wert von 5,7 bis 6,2 ist der Formaldehydrestgehalt auf
3,3 % gefallen. Man beendet die Reaktion durch Kühlen.
Verbrauch an Amin: 68 g.
Nach Einengen am Wasserstrahlvakuum erhält man 358 g eines
2,9 % Wasser enthaltenden Gemisches mehrwertiger Alkohole,
Hydroxyaldehyde und Hydroxyketone mit einem Gehalt an reduzierenden Anteilen, berechnet als Glucose, von 46,4 %,
einer Viskosität bei 50°C von 3069 mPa.s und einer OH-Zahl
von 70,3.

Le A 18 370

Patentansprüche:

1. Verfahren zur Herstellung von niedermolekularen Polyhydroxylverbindungen durch Selbstkondensation von Formaldehydhydrat in Gegenwart von löslichen oder unlöslichen
Verbindungen von Metallen der zweiten bis vierten Hauptgruppe oder der zweiten bis achten Nebengruppe des periodischen Systems der Elemente als Katalysator sowie gegebenenfalls in Gegenwart von zur Endiolbildung befähigten
Verbindungen als Cokatalysator und/oder von niedermolekularen und/oder höhermolekularen Polyhydroxylverbindungen, dadurch gekennzeichnet, daß der pH-Wert während der
Kondensationsreaktion durch die Zugabe von 5 bis 200 Milliäquivalenten eines tertiären Amins, bezogen auf 1 Mol
Formaldehyd, gesteuert wird.

2. Verfahren nach Anspruch 1, dadurch gekennzeichnet, daß
Amine eingesetzt werden, welche in ß-Stellung zum tertiären Stickstoffatom ein Elektronen anziehendes Heteroatom enthalten.

3. Verfahren nach Anspruch 1, dadurch gekennzeichnet, daß
Amine eingesetzt werden, welche in ß-Stellung zum tertiären Stickstoffatom eine Äthergruppe und/oder ein weiteres tertiäres Stickstoffatom enthalten.

4. Verfahren nach Anspruch 1, dadurch gekennzeichnet, daß als
tertiäres Amin N-Methylmorpholin, N,N'-Dimethylpiperazin
oder Anlagerungsprodukte mit einem Molekulargewicht bis
1000 von Äthylenoxid und/oder Propylenoxid an Morpholin
oder Piperazin eingesetzt werden.

Le A 18 370

| Kategorie | Kennzeichnung des Dokuments mit Angabe, soweit erforderlich, der maßgeblichen Teile | betrifft Anspruch |
|---|---|---|
| | <u>GB - A - 783 458</u> (CELANESE)<br><br>* Anspruch 1 *<br><br>— | 1 |
| DP | <u>DE - A - 2 639 084</u> (BAYER)<br><br>* Seite 26, letzter Abschnitt; Seite 27, erster Abschnitt; Patentanspruch 1; Seite 2, besonders Zeilen 1-3 *<br><br>— | 1 |
| DE | <u>DE - A - 2 714 084</u> (BAYER)<br><br>* Seite 29, letzter Abschnitt; Seite 30, beide ersten Abschnitte *<br><br>— | 1 |

**EINSCHLÄGIGE DOKUMENTE**

**KLASSIFIKATION DER ANMELDUNG (Int.Cl.²)**

C 07 H    3/04
            3/02
C 07 C  49/17
          47/19
          47/10
          31/18
          29/14
          29/00 //
C 08 G  18/42
          18/48

**RECHERCHIERTE SACHGEBIETE (Int. Cl.²)**

C 07 H    3/02
            3/04
C 07 C  49/17
          47/19
          47/10
          31/18
          29/14
          29/00

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 28-11-1978 | STOOS |

EPA form 1503.1   06.78